# EUROPEAN PATENT SPECIFICATION

(11) **EP 0 784 706 B1**
(45) Date of publication and mention of the grant of the patent: **01.12.1999**
(21) Application number: 95932641.4
(22) Date of filing: 22.09.1995
(51) Int. Cl.: C14C 1/04, C14C 1/06, C14C 1/08, C14C 1/00, C12S 7/00

(54) **A METHOD FOR PROCESSING OF HIDES OR SKINS INTO LEATHER, COMPRISING ENZYMATIC TREATMENT OF THE HIDE OR SKIN WITH A TRYPSIN ACTING MICROBIAL PROTEASE**
VERFAHREN ZUR LEDERHERSTELLUNG BEINHALTEND EINE ENZYMATISCHE BEHANDLUNG DER HAUTBLÖSSE MIT EINER TRYPSINAKTIVEN MIKROBIELLEN PROTEASE
PROCEDE DE TANNERIE PAR TRAITEMENT DES CUIRS ET PEAUX COMPORTANT UN TRAITEMENT ENZYMATIQUE DES CUIRS ET PEAUX AU MOYEN D'UNE PROTEASE MICROBIENNE A ACTION TRYPSINE

(30) Priority: 07.10.1994 DK 116494
(43) Date of publication of application: 23.07.1997
(73) Proprietor: NOVO NORDISK A/S, 2880 Bagsvaerd (DK)
(72) Inventor: SORENSEN, Niels, Henrik, DK-2880 Bagsvaerd (DK)
(86) International application number: DK9500380
(87) International publication number: WO9611285

(56) References cited:
- EP-A- 0 335 023
- WO-A-94/25583
- WO-A-95/00636
- GB-A- 1 172 055
- DERWENT'S ABSTRACT, No. 94-080995/10; & RU,C,2 002 806, 15.11.93.
- STN INTERNATIONAL, File CAPLUS, CAPLUS Accession No. 1976:448298, ORLITA, ALOIS, "Proteolytic Bacterial Enzymes - Effects of Bating on Them"; & REV. TECH. IND. CUIR, (1976), 68(3), 76-9.
- STN INTERNATIONAL, File CAPLUS, CAPLUS Accession No. 1993:554811, RYPNIEWSKI W.R. et al., "The Sequence and X-Ray Structure of the Trypsin from Fusarium Oxysporum"; & PROTEIN ENG., (1993), 6(4), 341-8.
- STN INTERNATIONAL, File CAPLUS, CAPLUS Accession No. 1992:23309, KOCHETOVA S.P. et al., "Effect of Fungal Alkaline Protease on Soaking-Tanning Processes"; & KOZH. OBUV. PROM-STI, M. (1990), 34-40.

## Description

### TECHNICAL FIELD

The present invention relates to a method for the processing of hides and skins into leather. More specifically, the present invention relates to a method for the processing of hides or skins into leather comprising enzymatic treatment of the hide or skin with a trypsin acting microbial protease.

### BACKGROUND ART

The traditional use of enzymes in a process for manufacturing leather is in the steps of soaking, unhairing and bating. In these processes, proteases are used to achieve a partial degradation of the proteins, thus making the pelt soft, supple and ready for subsequent tannage.

Proteases attack proteins and cut them into small soluble chains. When hides and skins are treated with proteases, the interfibrillary proteins and the proteoglucans covering the collagen fibrils are removed.

Different proteases have different actions and optimum conditions. Traditionally, the digestive enzymes, in particular trypsin and chymotrypsin, extracted from the pancreatic glands of slaughtered animals, usually pigs, have found use in the manufacture of leather.

Trypsin is the ideal proteolytic enzyme for use in bating. It is a highly specific proteolytic enzyme, acting on the lysyl and arginyl bonds of peptide chains only. Trypsin is regarded the most safe enzyme for use in the manufacture of leather.

The fibrillar molecules that make out the leather are collagen of types 1 and 3. In the processing of hides and skins into leather it is important not to damage this fibre structure excessively. Elastin is another fibrous type of protein, it is especially abundant in the regions in and just below the grain (the uppermost part). Its presence confers rigidity to the leather. For the production of certain types of leather, for instance like upholstery and garment leather, the removal of elastin can be seen as beneficial as the leather becomes softer. But in other types of leather (e.g. shoe upper leather) a certain amount of rigidity must be retained.

Pure trypsin is known not to break down native collagen, neither does it hydrolyse elastin. Therefore, pancreatins with a high trypsin content are often preferred in order to maintain rigidity and confer what is called "stand" to the shoe upper leather.

Trypsin appears as a byproduct from the insulin extraction from porcine pancreatic glands, a process which would be economically prohibitive if not insulin was of primary interest. However, developments within genetical engineering have now enabled the production of recombinant insulin. These developments have resulted in a scarcity of trypsin preparations and increasing prices.

These developments have initiated investigations to replace trypsin preparations with microbial protease preparations in the enzymatic processing of hides and skins into leather.

Proteolytic enzymes hitherto suggested for use in the manufacture of leather are proteases of bacterial origin derived from *Bacillus,* and proteases of fungal origin derived from *Aspergillus, Streptomyces, Microsporum,* and *Penicillium.*

However, none of these microbial proteases sufficiently mimic the action of trypsin, and no microbial protease has hitherto been able to fully replace trypsin in the processing of hides and skins into leather.

### SUMMARY OF THE INVENTION

It is an object of the present invention to provide an enzymatic process for the preparation of leather, which process comprises the action of a microbial protease able to mimic the action of trypsin.

Accordingly, in its first aspect, the present invention provides a method for the processing of hides or skins into leather, comprising enzymatic treatment of the hide or skin with a trypsin acting microbial protease.

In another aspect, the invention provides a bate comprising a trypsin acting microbial protease and suitable excipients.

### DETAILED DISCLOSURE OF THE INVENTION

The present invention provides a method for the processing of hides or skins into leather, comprising enzymatic treatment of the hide or skin with a trypsin acting microbial protease.

### The processing of hides or skins into leather

The hides and skins are usually received in the tanneries in the form of salted or dried raw hides or skins. The processing of hides or skins into leather comprises several different process steps including the steps of soaking, unhairing and bating. These steps constitute the wet processing and are performed in the beamhouse. Enzymatic treatment according to the present invention may take place any time during the manufacture of leather. However, proteases are usually employed during the wet processing, i.e. during soaking, unhairing and/or bating.

In one specific embodiment of the invention, the enzymatic treatment with a trypsin acting microbial protease takes place during the wet processing.

The object of the soaking step is to restore lost moisture to the salted and dried skins. In a more specific embodiment of the invention, the enzymatic treatment with a trypsin acting microbial protease takes place during soaking. A soaking process of the present invention may be performed at conventional soaking conditions, i.e. a pH in the range oH 6-11, preferably the range pH 7-10, a temperature in the range 20-30°C, preferably the range 24-28°C, and a reaction time in the range 2-24 hours, preferably the range 4-16 hours, and together with known tensides and preservatives, if needed.

The object of the bating step is to remove residual unhairing chemicals and non-leather making substances. The first phase of the bating step is termed deliming, during which phase lime and alkaline chemicals are eliminated.

The second phase of the bating step usually commences with the addition of the bate itself. In another specific embodiment, the enzymatic treatment takes place during bating. In a most preferred embodiment, the enzymatic treatment takes place during bating, after the deliming phase. A bating process of the present invention may be performed at conventional conditions, i.e. a pH in the range pH 6-9, preferably the range pH 6.5-8.5, a temperature in the range 20-30°C, preferably the range 25-28°C, and a reaction time in the range 20-90 minutes, preferably the range 40-80 minutes.

Processes for the manufacture of leather are well known to the person skilled in the art and have been described in e.g. WO 94/06942, WO 90/12118, US 3840433, EP-A1-505920, GB-A 2233665 and US 3986926.

### Trypsin acting microbial proteases

The process of the present invention comprises enzymatic treatment of the hide or skin with a trypsin acting microbial protease.

In the context of this invention, a trypsin acting microbial protease is a proteolytic enzyme able to mimic the action of trypsin, in particular when subjected to hides or skin under the conditions of a leather manufacturing process. The trypsin acting microbial protease of the invention preferably is a lysyl and/or arginyl specific proteolytic enzyme (endopeptidase), *i.e.* an enzyme capable of cleaving peptide bonds at the C-terminal side of lysine and/or arginine. The trypsin-like protease activity may e.g. be determined in an assay based on cleavage of a trypsin substrate such as N-Benzoyl-L-arginine-p-nitroanilide (L-BAPA or L-BAPNA).

Trypsin acting microbial proteases of fungal origin are known from *i.a. Aschersonia,* in particular *Aschersonia aleyrodis, Aspergillus, Beauvaria,* in particular *Beauvaria bassiana, Fusarium,* in particular *Fusarium oxysporum, Fusarium merismoides, Fusarium redolens, Fusarium sambucinum, Fusarium solani,* and *Fusarium verticilloides, Metarhizium,* in particular *Metarhizium anisopliae, Streptomyces,* in particular *Streptomyces griseus, Trichoderma* and *Verticillium,* in particular *Verticillium lecanii.* Thus EP 335,023 discloses trypsin acting fungal proteases obtained from *Aschersonia, Beauvaria, Metarhizium* and *Verticillium.*

In a preferred embodiment of the invention, the trypsin acting microbial protease is one derived from the strain *Fusarium oxysporum* DSM 2672. Trypsin acting proteases derived from *Fusarium oxysporum* DSM 2672, has been described in WO 89/06270 and WO 94/25583.

Trypsin acting microbial proteases of bacterial origin are known from e.g. *Lysobacter,* in particular *Lysobacter enzymogenes,* and *Achromobacter,* in particular *Achromobacter lyticus.* Thus *Jekel et al. [Jekel P A, Weijer W J and Beintema J J,* Anal. Biochem. 1983 **134** 347-354] describe a lysine specific endopeptidase obtained from *Lysobacter.*

The trypsin acting microbial protease may be of serine, thiol, metal or aspartate type.

The trypsin acting microbial protease of the invention may be applied in concentrations conventionally employed in leather manufacturing processes. It is at present contemplated that, in particular during soaking and bating, respectively, the trypsin acting microbial protease may be added in an amount corresponding to from 0.1 to 100 µg active enzyme protein per g of hide, preferably from 1 to 25 µg of active enzyme protein per g of hide.

### A Bate comprising a Trypsin Acting Microbial Protease

A bate is an agent or an enzyme containing preparation comprising the chemically active ingredients for use in beamhouse processes, in particular in the bating step of a process for the manufacture of leather.

In another aspect, the invention provides a bate comprising a trypsin acting microbial protease and suitable excipients. In the context of this invention, suitable excipients include the auxiliary chemicals known and used in the art, e.g. diluents, emulgators, delimers and carriers. The bate may be formulated as known and described in the art, e.g. as described in GB-A 2250289.

The bate of the invention may contain of from 0.00005 to 0.01 g of active enzyme protein per g of bate, preferably of from 0.0002 to 0.004 g of active protein per g of bate.

In a preferred embodiment, the bate of the present invention comprises a trypsin acting microbial protease which is derived from a strain of *Fusarium.* In a more preferred embodiment, the bate of the present invention comprises a trypsin acting microbial protease which is derived from the strain *Fusarium oxysporum* DSM 2762, or a mutant thereof.

### Proteolytic Activity

Proteolytic activity may be determined with denatured haemoglobin as substrate. In the Anson-Haemoglobin method for the determination of proteolytic activity, denatured haemoglobin is digested, and the undigested haemoglobin is precipitated with trichloroacetic acid (TCA). The amount of TCA soluble product is determined with phenol reagent, which gives a blue colour with tyrosine and tryptophan.

One Anson Unit (AU) is defined as the amount of enzyme which under standard conditions (i.e. 25°C, pH 7.5 and 10 min. reaction time) digests haemoglobin at an initial rate so that there is liberated per minute an amount of TCA soluble product which gives the same colour with phenol reagent as one milli-equivalent of tyrosine.

Determination of Anson Units and the proteolytic activity expressed by the Anson Units are concepts known by the person skilled in the art. However, a folder, AF 4/5, describing the analytical method in more detail is available upon request to Novo Nordisk A/S, Denmark, which folder is hereby included by reference.

### EXAMPLES

The invention is further illustrated with reference to the following examples, which are not intended to be in any way limiting to the scope of the invention as claimed.

### Example 1

In this example, the action of a trypsin acting microbial protease is evaluated at three different dosages of the enzyme.

The trypsin acting microbial protease used in this example is a *Fusarium oxysporum* protease, obtained according to WO 94/25583.

Three dry salted Danish dark/white Friesian bull hides of a total of 81 kg were treated according to the below recipe. All dosages are calculated as percentage on weight of the salted dry hides. The operations are carried out in thermostated pilot tanning drums.

### Recipe 1

| Dosage | Temp. °C | Product | pH | Time h, min |
|---|---|---|---|---|
| | | Wash | | |
| 200 | 20-25 | Water | | |
| | | Three hides | | |
| | | wash | | |
| | | | | 1 h |
| | | Drain | | |
| | | Soaking | | |
| 110 | 20-25 | Water | | |
| 0.1 | | Baymol™ AN | | |
| 0.01 | | Arazit™ KF | | |
| 0.3 | | Soda | 9.5-10 | 2 h |
| | | 10'/h | | over night |
| | | Drain | | |
| | | Liming | | |
| 1.0 | | NaHS | | |
| 1.5 | | NaS | | 15 min |
| 3 | | Lime | | |
| 100 | 25-28 | Water | | 120 min |
| | | 3'/30' | | over night |
| | | Drain | | |
| | | Wash | | |
| 200 | 20-25 | Water | | 10 min |
| | | Wash | | 10 min |

The pelts are taken out for fleshing and splitting. After splitting the pelts are parted along the backbone in two halves and weighed separately.

From pelt 1 the first half is treated without enzyme and the second half is treated with 3.6 µg of active trypsin-like enzyme protein per g of pelt.

From pelt 2 the first half is treated with 3.6 µg of active trypsin-like enzyme protein per g of pelt, while the second half is treated with 21 µg of active trypsin-like protein per g of pelt.

From pelt 3 the first half is treated with 21 µg of active trypsin-like enzyme protein per g of pelt, whereas the second half is treated without enzyme.

This system of treatments allows comparisons of dosages within the same pelt.

The halves are treated two and two together according to the following recipe.

### Recipe 2

| Dosage % | Temp. °C | Product | pH | Time h, min |
|---|---|---|---|---|
| | | Wash | | |
| 200 | 30 | Water | | 10 min |
| | | Drain | | |
| 200 | 35 | Water | | 10 min |
| | | Drain | | |
| | | Deliming | | |
| 150 | 35 | Water | | |
| 1.1 | | Decatal™ ES Fl. | | |
| 0.5 | | Decaltal™ R | | 30 min |
| | | | Check with Phenolphthalein pH 7.5 | |
| 150 | 30 | Water | | |
| As described in the text | | Enzyme | | 1 h |
| | | Drain | | |
| 200 | 30 | Water | | 10 min |
| | | Drain | | |
| 200 | 30 | Water | | 10 min |
| | | Drain | | |
| | | Stop bating by putting hide into a drum with a pickle float | | |

After bating the hides are collected in a drum with a pickle float, and after all the batings have been finished the pelts are treated all together according to the following recipe.

### Recipe 3

| | | Pickling | | |
|---|---|---|---|---|
| 70 | 30 | Water | | |
| 8 | | Salt | | 15 min |
| 0.25 | | HCOOH (96%) | | 10 min |
| 1.5 | | H₂SO₄(96%) | | 1 h |
| | | Check pH in hide | Acid | |
| 3.5 | | Chromosal™ B | | 15 min |
| 2.4 | | Baychrom™ CP | | over |
| | | | | night |
| | | Check pH drain and wash | around 4 | |

The resulting product, called "wet blue", is pressed to remove excess water and afterwards shaved to a thickness of 1.1 to 1.2 mm.

The wet blue was processed further into crust leather as follows.

### Recipe 4

| Dosage % | Temp. °C | Product | pH | Time h, min |
|---|---|---|---|---|
| | | Wet blue | | |
| 200 | 40 | Water | | 10 min |
| | | Drain | | |
| 150 2.0 2.0 | 40 | Water Chromosal™B Neutrigan™ F | 4.3 | 60 min |
| 2.0 0.65 | | Eskatan™ GLH Neutrigan™ MO | | 30 min |
| 2.0 | | Basyntan™ ANF | 5.0 6.3 | 60 min o.n. |
| | | Drain and wash | | |
| 200 | 40 | Water | | 10 min |
| | | Drain | | |
| 100 3.0 | 40 | Water Tamol™ PM | | 10 min |
| 3.0 | | Avalan beige™ LGG | | 45 min |
| | | Fat liquoring | | |
| 100 more | 55 | Water Cutapol" TIS | | 45 min |
| 4.0 | | Basyntan™ ANF | 6.4 | 45 min |
| 6.5 | | HCOOH (20%) | | 15 min |
| 6.5 | | HCOOH (20%) | | 30 min |
| | | Drain and wash with cold water | | |

The leather was air dried.

The visual inspection of the beige coloured crust leathers after drying showed that compared to the untreated sides, the trypsin-like enzyme treated sides had much cleaner and lighter coloured grain with less wrinkles. The enzyme treated sides were 6 to 10% longer than the non-treated sides due to the leather being more relaxed and less wrinkly. Also did the trypsin-like enzyme treatment increase the tear strength 20 to 25% compared to the non-treated, when analyzed according to DIN 53 329.

Colour measurements using a Minolta™ Chroma Meter showed that comparing within hides, the trypsin-like enzyme treated sides were significantly lighter in colour (higher L-star value) than no-treated sides, which is a result of the enzyme treated sides being cleaner than the untreated. There were found no significant differences between the two enzyme dosages.

During the visual inspections and using microscopy, no damages to the grain were observed, whatsoever, not even at the high 21 µg treatments.

### Example 2

In this example, the action of a trypsin-like protease obtained from Fusarium is compared with the actions of a pure trypsin bate (PTN™ Crystalline, available from Novo Nordisk A/S, Denmark) and a pancreatin bate (PTN™ 3.0S, available from Novo Nordisk A/S, Denmark), respectively, in a bating process.

The trypsin-like microbial protease used in this example is a *Fusarium oxysporum* protease, obtained according to WO 94/25583.

Two black white Friesian bull hides are used in this example which is run in two series. The hides are processed together in a pilot drum according to Recipe 1 of Example 1. Just before bating, each hide is cut in two halves and treated according to Recipe 5, below. The halves are bated separately as follows (before enzyme treatment they have been delimed):

**Table 1**

| Pelt No. | First half (A) | Second half (B) |
|---|---|---|
| 1 | 0.0541% Trypsin-like | 0.00044% PTN™ Crystalline |
| | | |
| 2 | 0.2937% Trypsin-like | 0.0086% PTN™ 3.0S |

Different treatment is then applied on the same pelt ensuring the possibility of comparing effects. All dosages are calculated as percentage on weight of the limed, fleshed and splitted pelt. The protease dosage on pelt 1 is equal to 3 µg of active enzyme protein per g of pelt, and the protease dosage on pelt 2 is equal to 3.1 x 10⁻⁴ Anson Units per g of pelt.

### Recipe 5

| Dosage % | Temp. °C Product | Wash | pH | Time h, min |
|---|---|---|---|---|
| 200 | 30 | Water | | 10 min |
| | | Drain | | |
| 200 | 35 | Water | | 10 min |
| | | Drain | | |
| | | Deliming | | |
| 150 | 35 | Water | | |
| 2.0 | | Purgamin™ GSB Neu | 7.5 Check with Phenolphthalein | 45 min |
| | | Bating | | |
| 150 | 30 | Water | | |
| As described above in text | | Enzyme | | 1 hr |
| | | Drain | | |
| 70 | 20 | Water | | |
| 9 | | Sodium chloride | | 5 min |
| 0.25 | | HCOOH(96%) | | |
| 1.5 | | H₂SO₄(96%) | 2.5 | 1 hr |
| 3.5 | | Chromosal™ B | | |
| 2.4 | | Baychrome™ CP | | overnight |

The resulting wet blue is then pressed to remove excess water and afterwards shaved to a thickness of 1.1 to 1.2 mm. Thereafter the wet blue is processed into crust leather according to Recipe 4 of Example 1.

Then the leather was air dried.

Evaluations on bated pelt and wet blues did not disclose any significant differences between treatments. Neither did we find any systematic differences between proteases evaluating the crust leather visually. The sides were all much alike.

Samples of leather were cut out and measured for tear strength (DIN 53329), tensile strength (DIN 53328), lastometer test (DIN 53325) and flexometer test (DIN 53351), respectively. The results appear from tables 2 and 3 below.

The lastometer test, giving the grain crack and burst figures, is a test for the expandability of the grain. It is measured on an apparatus in which a ball-ended rod is forced against the centre of a diaphragm of the material until it cracks and bursts. The force and distension are measured.

The tensile strength is measured as the strength and the extension at break and the tear strength is the force applied to tear the leather both perpendicularly and parallelly to the fibre orientation in the leather.

The flexometer tests how leather responds to bending forces (like the lastometer test this test is especially oriented towards the shoe upper leather testing). The leather is folded in a defined way and exposed to repeated flexing to and fro in a Bally Flexometer. The flexometer test is not, however, included in the below tables, as no cracks were observed on any of the leathers after 50,000 dr flexes.

**Table 2**

| Trypsin-like (1A) contra PTN™ Crystalline (1B): | | | | | |
|---|---|---|---|---|---|
| Sides | Grain crack load kg | Grain burst load kg | Tensile strength N/mm² | % Extension at break | Tear strength N/mm² |
| 1A | 14 | 24 | 12 | 78 | 46 |
| 1B | 14 | 26 | 15 | 97 | 65 |
| Difference | 0% | 8% | 25% | 24% | 41% |

**Table 3**

| Trypsin-like (2A) contra PTN™ 3.0S (2B): | | | | | |
|---|---|---|---|---|---|
| Sides | Grain crack load kg | Grain burst load kg | Tensile strength N/mm² | % Extension at break | Tear strength N/mm² |
| 2A | 10 | 21 | 10 | 79 | 36 |
| 2B | 14 | 24 | 8 | 76 | 34 |
| Difference | 40% | 14% | -20% | -4% | -6% |

In order to interpret the data of tables 2-3 it is essential not to look on each figure only, but to take an overall view of the data. First of all, there is a large hide to hide variation, but even though the leather samples have been taken from the same or parallel area on the leather sides, there is of course still some natural variation in the leather from side to side within the same hide.

Taking this into consideration, PTN™ Crystalline seems to have the highest performance overall, whereas PTN™ 3.0S and the trypsin-like protease of the invention are close.

This example demonstrates that the trypsin acting protease according to the invention is an excellent microbial alternative to the highly purified pancreatin and trypsin bates.

## Claims

1. A method for the processing of hides or skins into leather, comprising enzymatic treatment of the hide or skin with a trypsin acting microbial protease.

2. The method according to claim 1, in which the trypsin acting microbial protease is of fungal origin.

3. The method according to claim 2, in which the trypsin acting fungal protease is derived from a strain of *Aschersonia,* a strain of *Fusarium,* a strain of *Metarhizium,* a strain of *Trichoderma,* or a strain of *Verticillium.*

4. The method according to claim 3, in which the trypsin acting fungal protease is derived from a strain of *Aschersonia aleyrodis,* a strain of *Fusarium oxysporum, Fusarium merismoides, Fusarium redolens, Fusarium sambucinum, Fusarium solani,* and *Fusarium verticilloides,* a strain of *Metarhizium anisopliae,* or a strain of *Verticillium lecanii.*

5. The method according to claim 4, in which the trypsin acting fungal protease is derived from the strain *Fusarium oxysporum* DSM 2672, or a mutant or a variant thereof.

6. The method according to claim 1, in which the trypsin acting microbial protease is of bacterial origin.

7. The method according to claim 6, in which the trypsin acting bacterial protease is derived from a strain of *Achromobacter,* preferably *Achromobacter lyticus,* or a strain of *Lysobacter,* preferably *Lysobacter enzymogenes.*

8. The method according to any of claims 1-7, in which the trypsin acting microbial protease is a serine protease.

9. The method according to any of claims 1-8, in which the trypsin acting microbial protease is a Lys and/or Arg specific protease.

10. The method according to any of claims 1-9, in which the enzymatic treatment is accomplished during one or more of the wet processing steps.

11. The method according to claim 10, in which the enzymatic treatment is accomplished during the soaking step.

12. The method according to claim 10, in which the enzymatic treatment is accomplished during the bating step.

13. A bate comprising a trypsin acting microbial protease and suitable excipients.

14. The bate according to claim 13, in which the trypsin acting microbial protease is of fungal origin, preferably derived from a strain of *Aschersonia,* a strain of *Fusarium,* a strain of *Metarhizium,* a strain of *Trichoderma,* or a strain of *Verticillium.*

15. The bate according to claim 14, in which the trypsin acting fungal protease is derived from a strain of *Aschersonia aleyrodis,* a strain of *Fusarium oxysporum, Fusarium merismoides, Fusarium redolens, Fusarium sambucinum, Fusarium solani,* and *Fusarium verticilloides,* a strain of *Metarhizium anisopliae,* or a strain of *Verticillium lecanii.*

16. The bate according to claim 15, in which the trypsin acting fungal protease is derived from the strain *Fusarium oxysporum* DSM 2672, or a mutant or a variant thereof.

17. The bate according to claim 13, in which the trypsin acting microbial protease is of bacterial origin.

18. The bate according to claim 17, in which the trypsin acting bacterial protease is derived from a strain of *Achromobacter,* preferably *Achromobacter lyticus,* or a strain of *Lysobacter,* preferably *Lysobacter enzymogenes.*

## Patentansprüche

1. Verfahren zur Verarbeitung von Häuten oder Fellen zu Leder, umfassend die enzymatische Behandlung der Haut oder des Fells mit einer mikrobiellen Protease mit Trypsinwirkung.

2. Verfahren nach Anspruch 1, wobei die mikrobielle Protease mit Trypsinwirkung aus Pilzen stammt.

3. Verfahren nach Anspruch 2, wobei die Pilz-Protease mit Trypsinwirkung von einem Stamm von *Aschersonia,* einem Stamm von *Fusarium,* einem Stamm von *Metarhizium,* einem Stamm von *Trichoderma* oder einem Stamm von *Verticillium* stammt.

4. Verfahren nach Anspruch 3, wobei die Pilz-Protease mit Trypsinwirkung von einem Stamm von *Aschersonia aleyrodis,* einem Stamm von *Fusarium oxysporum, Fusarium merismoides, Fusarium redolens, Fusarium sambucinum, Fusarium solani* und *Fusarium verticilloides,* einem Stamm von *Metarhizium anisopliae* oder einem Stamm von *Verticillium lecanii* stammt.

5. Verfahren nach Anspruch 4, wobei die Pilz-Protease mit Trypsinwirkung vom Stamm *Fusarium oxysporum* DSM 2672 oder von einer Mutante oder Variante davon stammt.

6. Verfahren nach Anspruch 1, wobei die mikrobielle Protease mit Trypsinwirkung bakteriellen Ursprungs ist.

7. Verfahren nach Anspruch 6, wobei die bakterielle Protease mit Trypsinwirkung von einem Stamm von *Achromobacter,* vorzugsweise *Achromobacter lyticus,* oder von einem Stamm von *Lysobacter,* vorzugsweise *Lysobacter enzymogenes,* stammt.

8. Verfahren nach einem der Ansprüche 1-7, wobei die mikrobielle Protease mit Trypsinwirkung eine Serin-Protease ist.

9. Verfahren nach einem der Ansprüche 1-8, wobei die mikrobielle Protease mit Trypsinwirkung eine Lys- und/oder Arg-spezifische Protease ist.

10. Verfahren nach einem der Ansprüche 1-9, wobei die enzymatische Behandlung während einem oder mehreren der Naßverarbeitungsschritte durchgeführt wird.

11. Verfahren nach Anspruch 10, wobei die enzymatische Behandlung während des Wässerungsschrittes durchgeführt wird.

12. Verfahren nach Anspruch 10, wobei die enzymatische Behandlung während des Beizschrittes durchgeführt wird.

13. Beize, enthaltend eine mikrobielle Protease mit Trypsinwirkung und geeignete Hilfsstoffe.

14. Beize nach Anspruch 13, wobei die mikrobielle Protease mit Trypsinwirkung aus Pilzen stammt, vorzugsweise von einem Stamm von *Aschersonia,* einem Stamm von *Fusarium,* einem Stamm von *Metarhizium,* einem Stamm von *Trichoderma* oder einem Stamm von *Verticillium.*

15. Beize nach Anspruch 14, wobei die Pilz-Protease mit Trypsinwirkung von einem Stamm von *Aschersonia aleyrodis,* einem Stamm von *Fusarium oxysporum, Fusarium merismoides, Fusarium redolens, Fusarium sambucinum, Fusarium solani* und *Fusarium verticilloides,* einem Stamm von *Metarhizium anisopliae* oder einem Stamm von *Verticillium lecanii* stammt.

16. Beize nach Anspruch 15, wobei die Pilz-Protease mit Trypsinwirkung vom Stamm *Fusarium oxysporum* DSM 2672 oder einer Mutante oder Variante davon stammt.

17. Beize nach Anspruch 13, wobei die mikrobielle Protease mit Trypsinwirkung bakteriellen Ursprungs ist.

18. Beize nach Anspruch 17, wobei die bakterielle Protease mit Trypsinwirkung von einem Stamm von *Achromobacter, vorzugsweise Achromobacter lyticus,* oder einem Stamm von *Lysobacter,* vorzugsweise *Lysobacter enzymogenes,* stammt.

## Revendications

1. Procédé de traitement de cuirs ou de peaux en un article de mégisserie, comportant un traitement enzymatique du cuir ou de la peau à l'aide d'une protéase microbienne agissant comme une trypsine.

2. Procédé selon la revendication 1, dans lequel la protéase microbienne agissant comme une trypsine est d'origine fongique.

3. Procédé selon la revendication 2, dans lequel la protéase fongique agissant comme une trypsine est dérivée d'une souche de *Aschersonia,* d'une souche de *Fusarium,* d'une souche de *Metarhizium,* d'une souche de *Trichoderma,* ou d'une souche de *Verticillium.*

4. Procédé selon la revendication 3, dans lequel la protéase fongique agissant comme une trypsine est dérivée d'une souche de *Aschersonia aleyrodis,* d'une souche de *Fusarium oxysporum, Fusarium merismoides, Fusarium redolens, Fusarium sambucinum, Fusarium solani,* et *Fusarium verticilloides,* d'une souche de *Metarhizium anisopliae,* ou d'une souche de *Verticillium lecanii.*

5. Procédé selon la revendication 4, dans lequel la protéase fongique agissant comme une trypsine est dérivée de la souche DSM 2672 de *Fusarium oxysporum,* ou d'un mutant ou d'un variant de celle-ci.

6. Procédé selon la revendication 1, dans lequel la protéase microbienne agissant comme une trypsine est d'origine bactérienne.

7. Procédé selon la revendication 6, dans lequel la protéase bactérienne agissant comme une trypsine est dérivée d'une souche de *Achromobacter,* de préférence *Achromobacter lyticus,* ou d'une souche de *Lysobacter,* de préférence *Lysobacter enzymogenes.*

8. Procédé selon l'une quelconque des revendications 1 à 7, dans lequel la protéase microbienne agissant comme une trypsine est une protéase à sérine.

9. Procédé selon l'une quelconque des revendications 1 à 8, dans lequel la protéase microbienne agissant comme une trypsine est une protéase spécifique à Lys et/ou Arg.

10. Procédé selon l'une quelconque des revendications 1 à 9, dans lequel le traitement enzymatique est effectué pendant une ou plusieurs étapes de traitement par voie humide.

11. Procédé selon la revendication 10, dans lequel le traitement enzymatique est effectué pendant l'étape de trempage.

12. Procédé selon la revendication 10, dans lequel le traitement enzymatique est effectué pendant l'étape de confitage.

13. Confit comportant une protéase microbienne agissant comme une trypsine et des excipients adaptés.

14. Confit selon la revendication 13, dans lequel la protéase microbienne agissant comme une trypsine est d'origine fongique, de préférence dérivée d'une souche de *Aschersonia,* d'une souche de *Fusarium,* d'une souche de *Metarhizium,* d'une souche de *Trichoderma,* ou d'une souche de *Verticillium.*

15. Confit selon la revendication 14, dans lequel la protéase fongique agissant comme une trypsine est dérivée d'une souche de *Aschersonia aleyrodis,* d'une souche de *Fusarium oxysporum,* de *Fusarium merismoides,* de *Fusarium redolens,* de *Fusarium sambucinum,* de *Fusarium solani,* et de *Fusarium verticilloides,* d'une souche de *Metarhizium anisopliae,* ou d'une souche de *Verticillium le*canii.

16. Confit selon la revendication 15, dans lequel la protéase fongique agissant comme une trypsine est dérivée de la souche DSM 2672 de *Fusarium oxysporum,* ou d'un mutant ou d'un variant de celle-ci.

17. Confit selon la revendication 13, dans lequel la protéase microbienne agissant comme une trypsine est d'origine bactérienne.

18. Confit selon la revendication 17, dans lequel la protéase bactérienne agissant comme une trypsine est dérivée d'une souche de *Achromobacter,* de préférence *Achromobacter lyticus,* ou d'une souche de *Lysobacter,* de préférence *Lysobacter enzymogenes.*
